# EUROPÄISCHE PATENTSCHRIFT

(11) **EP 0 634 391 B1**
(45) Veröffentlichungstag und Bekanntmachung des Hinweises auf die Patenterteilung: **22.10.1997**
(21) Anmeldenummer: 94110393.9
(22) Anmeldetag: 04.07.1994
(51) Int. Cl.: C07C 209/36

(54) **Kontinuierliches Verfahren zur Herstellung von aromatischen Aminen**
Continuous process for the preparation of aromatic amines
Procédé en continu de préparation d'amines aromatiques

(30) Priorität: 15.07.1993 DE 4323687
(43) Veröffentlichungstag der Anmeldung: 18.01.1995
(73) Patentinhaber: BAYER AG, 51368 Leverkusen (DE)
(72) Erfinder: Zarnack, Uwe Jens, Dr., D-25541 Brunsbüttel (DE); Pohl, Fritz, Dr., D-25541 Brunsbüttel (DE); Grenner, Dieter, Dr., D-51373 Leverkusen (DE); Hetzel, Hartmut, Dr., D-50858 Köln (DE); Judat, Helmut, Dr., D-40764 Langenfeld (DE)

(56) Entgegenhaltungen:
- EP-A- 0 124 010
- EP-A- 0 223 035
- EP-A- 0 263 935
- DD-A- 229 940

## Beschreibung

Die Erfindung betrifft ein neues kontinuierliches Verfahren zur Herstellung von aromatischen Di- und Polyaminen durch katalytische Hydrierung der den Aminen entsprechenden Di- und Polynitroverbindungen bei hohen Temperaturen und gegebenenfalls gleichzeitiger Wärmeabfuhr aus dem Reaktionsgemisch zur Erzeugung von Dampf mit einem Überdruck von ≥ 2 bar.

Verfahren zur Herstellung von aromatischen Aminen durch katalytische Hydrierung der ihnen zugrundeliegenden Nitroverbindungen sind in großer Zahl bekannt (US 3 546 296, US 3 781 373, US 3 761 521, US 3 895 065, US 4 288 640, DE 2 135 154, US 3 882 048, GB 1 490 313, GB 1 017 646, US 3 356 728, US 3 356 729, US 3 431 085, US 3 194 839, EP-A-0 223 035 und GB 768 111).

Bei der Umsetzung von aromatischen Polynitroverbindungen mit Wasserstoff wird eine beträchtliche Wärmemenge frei. Es hat nicht an Versuchen gefehlt, die bei der Herstellung von aromatischen Polyaminen durch Hyrierung der entsprechenden Polynitroverbindung freiwerdende Hydrierwärme zu nutzen. So kann z.B. das erwärmte Kühlwasser zur Beheizung von Räumen oder zur Erwärmung von Produktströmen oder auch zur Verdampfung niedrig siedender Lösungsmittel eingesetzt werden.

Allgemein wird die großtechnische Hydrierung von aromatischen Polynitroverbindungen jedoch bei möglichst niedrigen Temperaturen durchgeführt, da bei der Hydrierung von aromatischen Polynitroverbindungen bei hohen Temperaturen die Gefahr unkontrollierter Nebenreaktionen besteht. Diese Nebenreaktionen können zur Bildung von unerwünschten Nebenprodukten und damit zu Ausbeuteminderungen führen. Beispielhaft erwähnt seien in diesem Zusammenhang Kernhydrierungen, hydrogenolytische Spaltungen oder die Bildung von hochmolekularen, teerartigen Produkten. Es können auch explosionsartige Nebenreaktionen ablaufen, die im stark exothermen Reaktionsverlauf der Nitrogruppenumsetzung und ihrer hohen Reaktionsgeschwindigkeit bei höheren Temperaturen begründet sind.

Um diese unerwünschten Nebenreaktionen soweit wie möglich auszuschließen, wurde daher im allgemeinen die großtechnische Hydrierung von aromatischen Polynitroverbindungen vorwiegend bei solchen Temperaturen durchgeführt, die die Erzeugung von Dampf mit einem über 2 bar liegenden Überdruck nicht gestatten.

In CA 1 211 757 wird ein Verfahren zur Herstellung von aromatischen Diaminen durch katalytische Hydrierung der entsprechenden Dinitroverbindungen unter gleichzeitiger Erzeugung von Dampf mit einem Überdruck von ≥ 1 bar beschrieben. Als Reaktor wird eine mit Fieldrohren versehene Blasensäule verwendet und die Kühlung des Reaktors erfolgt mittels Wasser, das in den Fieldrohren in Dampf überführt wird. Eine Reaktionssuspension, die im wesentlichen aus einer aromatischen Dinitroverbindung, dem entsprechenden Diamin, einem Hydrierungskatalysator, einem gesättigten, aliphatischen Alkohol mit 1 bis 6 Kohlenstoffatomen als Lösungsmittel und Wasser besteht, wird in den Reaktor mit dem Wasserstoff eingespeist. Die Menge der in die Blasensäule eingespeisten Reaktionssuspension sowie der Druck, die Temperatur und die Menge des Kühlwassers werden so bemessen, daß in der Blasensäule eine Reaktionstemperatur zwischen 140 und 250°C vorliegt.

Nachteilig bei dem in CA 1 211 757 beschriebenen Verfahren ist die Verwendung eines Lösungsmittels, das zwar die bekannten Probleme der Hydrierung von Polynitroverbindungen bei höheren Temperaturen mildert, unter den Hydrierbedingungen jedoch nicht vollständig inert ist, was zu unerwünschten Nebenprodukten und Ausbeuterminderungen führt. Nach erfolgter Reaktion muß das Lösungsmittel außerdem vom aromatischen Diamin aufwendig abgetrennt und gegebenenfalls aufbereitet werden.

Es wurde deshalb versucht, wesentliche Merkmale des in CA 1 211 757 beschriebenen Verfahrens auf eine lösungsmittelfreie katalytische Hydrierung aromatischer Nitroverbindungen zu übertragen. In EP 263 935 werden Rührreaktoren zur Durchführung von exothermen Reaktionen beansprucht, die dadurch gekennzeichnet sind, daß die Kühlung der Reaktoren mittels Wasser erfolgt, das in Fieldrohren in Dampf von mehr als 1 bar Überdruck überführt wird. Das Verhältnis der Kühlfläche der Siederohre zum Volumen des Reaktionsraumes beträgt 40 bis 400 m²/m³. Dieses hohe Verhältnis hat sich als besonders effektiv für die Abfuhr der entstehenden Reaktionswärme erwiesen. Das beanspruchte Verfahren ist nur bedingt für die katalytische Hydrierung von Polynitroaromaten einsetzbar, da die Phasendurchmischung nicht sichergestellt ist. Aufgrund der Inhomogenitäten laufen verstärkt unkontrollierbare Nebenreaktionen unter Ausbeuteverminderung ab und die Kühlflächen belegen sich mit harzartigen Verbindungen und/oder Katalysatoranteilen.

Aufgabe war es daher, ein lösungsmittelfreies Verfahren zur Herstellung von aromatischen Aminen durch Hydrierung von aromatischen Nitroverbindungen zur Verfügung zu stellen, das es gestattet, bei hohen Temperaturen ohne Nebenreaktionen zu arbeiten und gegebenenfalls gleichzeitig einen Dampf mit einem Überdruck von ≥ 2 bar zu erzeugen.

Diese Aufgabe konnte durch das erfindungsgemäße Verfahren gelöst werden.

Gegenstand der Erfindung ist ein kontinuierliches Verfahren zur Herstellung von aromatischen Di- und Polyaminen durch katalytische Hydrierung der entsprechenden aromatischen Di- und Polynitroverbindungen mit Wasserstoff unter Verwendung von Hydrierungskatalysatoren, welches dadurch gekennzeichnet ist, daß in einem Loop-Venturi-Reaktor mit Ejektor
a) in ein schnell fließendes Gemisch, das im wesentlichen aus aromatischem Di- oder Polyamin, feinteilig suspendiertem, festem Hydrierungskatalysator, Wasser und Wasserstoff besteht, ohne Lösungsmittel die entsprechende aromatische Di- oder Polynitroverbindung so eingetragen wird, daß
   a1) die Di- oder Polynitroverbindung in dem Gemisch fein verteilt ist,
   a2) das volumetrische Verhältnis des umgepumpten, schnell fließenden Gemisches zur eingetragenen Di- oder Polynitroverbindung 50 bis 500 beträgt,
   a3) mit dem umgepumpten, schnell fließenden Gemisch der Ejektor so betrieben wird, daß im Mischraum des Ejektors eine Energie von 4 bis 40 kW/t eingetragener Di- oder Polynitroverbindung dissipiert wird,
   a4) und ein Wasserstoffvolumenstrom im Verhältnis zum umgepumpten Gemischvolumen von 0,1 bis 7 angesaugt wird, wobei der benötigte Wasserstoff dem Gasraum des Reaktors entzogen wird und der bei der Reaktion verbrauchte Wasserstoff an beliebiger Stelle des Systems nachgeliefert wird,
b) das Reaktionsgemisch kontinuierlich aus dem System ausgetragen wird,
c) im Reaktor ein Druck von 5 bis 100 bar und eine Betriebstemperatur von 120-220°C aufrechterhalten werden und
d) gegebenenfalls die Reaktionswärme zur Dampferzeugung genutzt wird.

Bevorzugt wird im Reaktor ein Druck von 10 bis 50 bar und eine Betriebstemperatur von 150 bis 200°C aufrechterhalten.

Der Produktaustrag aus dem System kann an beliebiger Stelle erfolgen.

Bevorzugt erfolgt der Austrag auf der Druckseite der Umwälzpumpe über eine Filtereinheit oder ohne eine solche.

Der Produktaustrag kann auch aus dem Reaktor selber erfolgen über eine Beruhigungszone oder ohne eine solche. In der Beruhigungszone kann der Katalysator z.B. durch Sedimentation vom Produkt abgetrennt und dann in das System zurückgeführt werden.

Bevorzugt erfolgt der Austrag unter Rückhalt des Katalysators.

Im erfindungsgemäßen Verfahren kann die aromatische Di- oder Polynitroverbindung in reiner Form, als Mischung mit dem entsprechendem Di- oder Polyamin oder als Mischung mit dem entsprechenden Di- oder Polyamin und Wasser eingesetzt werden.

Die aromatische Di- oder Polynitroverbindung wird in das Gemisch so eingetragen, daß sie hierin fein verteilt vorliegt. Bevorzugt kann dies über eine Ringlochdüse oder über andere geeignete Vorrichtungen erfolgen.

Vorzugsweise wird die Nitroverbindung in den Ejektor eingetragen, besonders bevorzugt in den Mischraum des Ejektors.

Beispiele bevorzugt eingesetzter aromatischer Nitroverbindungen sind: 1,3-Dinitrobenzol, 2,4-Dinitrotoluol, 2,6-Dinitrotoluol oder im wesentlichen aus den beiden letztgenannten Isomeren bestehende, technische Dinitrotoluolgemische.

Besonders bevorzugt werden als aromatische Nitroverbindungen 2,4-Dinitrotoluol oder dessen technische Gemische mit bis zu 35 Gew.-%, bezogen auf das Gesamtgemisch, an 2,6-Dinitrotoluol eingesetzt. Diese technischen Gemische können auch untergeordnete Mengen, d.h. bis max. 6 Gew.-%, bezogen auf das Gesamtgemisch, an 2,3-, 2,5- oder 3,4-Dinitrotoluol enthalten.

Für das erfindungsgemäße Verfahren werden die an sich bekannten Hydrierungskatalysatoren für aromatische Nitroverbindungen verwendet. Gut geeignet sind insbesondere die Metalle der 8. Nebengruppe des Periodensystems der Elemente, die beispielsweise auf Trägermaterialien wie Oxiden von Magnesium, Aluminium und/oder Silizium aufgebracht sein können. Vorzugsweise werden Raneyeisen, -kobalt und/oder -nickel, insbesondere Raneynickel, verwendet. Die Katalysatoren werden in feinverteiltem Zustand eingesetzt und liegen in der Reaktionssuspension feinteilig-suspendiert vor. Die Aufrechterhaltung dieser Suspension bei der Durchführung des erfindungsgemäßen Verfahrens wird durch die Umwälzung an der Ejektordüse gewährleistet.

Es ist bei der Durchführung des erfindungsgemäßen Verfahrens besonders darauf zu achten, daß
- die Umwälzung des Reaktionsgemisches so betrieben wird, daß das Volumenverhältnis des Gemisches zur eingetragenen Nitroverbindung 50 bis 500, vorzugsweise 200 bis 300 beträgt,
- die Energiedissipation an der Ejektordüse 4 bis 40 kW/t eingetragener Nitroverbindung beträgt,
- die Injektordüse selbstansaugend ist, d.h., daß sich das im oberen Reaktorteil sammelnde Wasserstoffgas selbständig durch die Energie des umgewälzten Gemisches wieder in das Reaktionsgemisch einmischt,
- der Betriebsdruck des Systems durch Zuführung von frischem Wasserstoff von außen aufrechterhalten wird und
- das volumetrische Verhältnis von angesaugtem Wasserstoffstrom zu umgepumptem Gemisch 0,1 bis 7 beträgt.

In Verbindung mit der Eindüsung der aromatischen Nitrokomponente (bevorzugt unmittelbar vor der Ejektordüse) wird mit den übrigen Verfahrensparametern eine intensive Durchmischung aller Komponenten bei niedrigen Substratkonzentrationen und unter Vermeidung alleiniger Substrat/Katalysator-Kontaktzeiten erzielt. Dadurch wird eine lösungsmittelfreie, katalytische Hydrierung von Di- und Polynitroaromaten bei hohen Temperaturen möglich, so daß durch Wärmeabfuhr aus dem System gleichzeitig Dampf von mehr als 2 bar Überdruck erzeugt werden kann. Nebenreaktionen oder dergleichen treten nicht oder nur untergeordnet auf.

Das erfindungsgemäße Verfahren wird bevorzugt in Apparaturen, wie sie in Chem. Eng. Sc., Vol. 47, No. 13/14, p. 3557-3564 (1992) oder in VT "Verfahrenstechnik" 15 (1981) Nr. 10, S. 738-749 beschrieben sind, durchgeführt. Auch andere Apparaturen, die in den wesentlichen Punkten mit den obengenannten Apparaturen übereinstimmen, können eingesetzt werden.

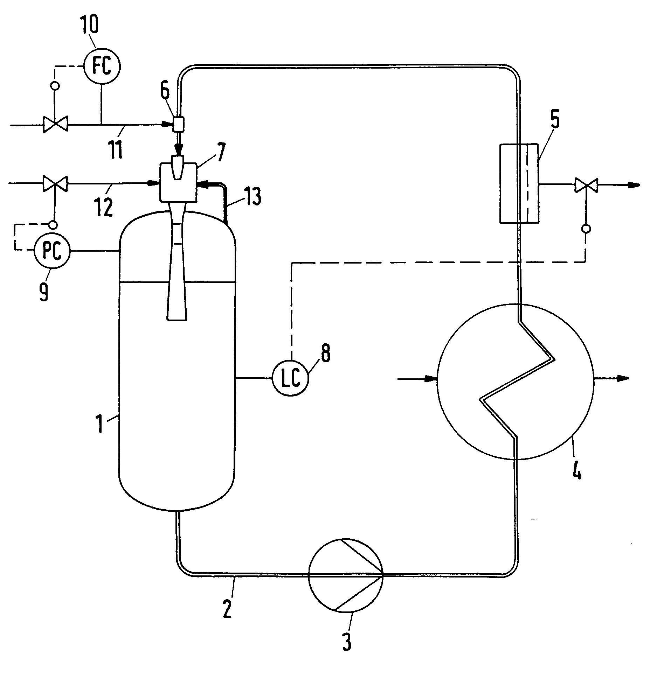

Das erfindungsgemäße Verfahren kann z.B. in einer Apparatur durchgeführt werden, die in der Abbildung schematisch dargestellt ist. In dieser Abbildung haben die Zahlen folgende Bedeutung:
(1) Behälter
(2) Rohrsystem für die Kreislaufführung des Reaktionsgemischs
(3) Pumpe zur Erzeugung des Reaktionsgemischkreislaufs
(4) Wärmetauscher zur Kühlung des umgewälzten Reaktionsgemischs
(5) Filtereinheit zum Austrag des aromatischen Amins unter Zurückhaltung des Katalysators
(6) Einspeisevorrichtung für die aromatische Nitroverbindung
(7) Ejektor zum Ansaugen des Wasserstoffs in das Reaktionsgemisch
(8) Standregelung
(9) Druckhaltung
(10) Mengenregelung für die aromatische Nitroverbindung
(11) Zuführungsleitung für die aromatische Nitroverbindung
(12) Zuführungsleitung für den Wasserstoff
(13) Wasserstoffüberleitung.

Im folgenden soll das erfindungsgemäße Verfahren näher erläutert werden:

Gemaß dem erfindungsgemäßen Verfahren kann z.B. die Hydrierung von Dinitrotoluol (DNT) in einem Schleifenreaktor mit Ejektor stattfinden. Die Reaktion wird bevorzugt bei einem Druck von 25 bar und einer Temperatur von 185°C durchgeführt und ist stark exotherm, d.h. die freiwerdende Reaktionswärme führt zu einer nicht unerheblichen Erwärmung des Reaktionsgemisches. Da die Selektivität sich mit steigender Temperatur verschlechtert, ist es vorteilhaft, die Temperaturerhöhung des Reaktionsgemisches auf 10°C oder weniger zu begrenzen. Dies kann in einfacher Weise dadurch geschehen, daß der Mengenstrom des umgepumpten Gemisches der freigesetzten Wärmemenge und der tolerierbaren Temperaturerhöhung angepaßt wird. Bei der oben beschriebenen Hydrierung von z.B. 2000 kg/h Dinitrotoluol wird eine Wärmemenge von 2.756.000 kcal/h frei. Die zulässige Maximal-Temperatur von 185°+10°=195°C wird nicht überschritten, wenn der Mengenstrom des umgepumpten Reaktionsgemisches mindestens 372 t/h beträgt. Bei einer zudosierten DNT-Menge von 1500 l/h ergibt sich somit ein volumetrisches Verhältnis von Menge Gemisch zu Menge DNT wie 372 000:1500=248:1. Bei Verwendung von Katalysatoren, die weniger temperaturempfindlich sind, können natürlich auch größere Temperaturerhöhungen zugelassen und mithin geringere Mengenströme umgepumpt werden.

Für die Durchführung der Reaktion wäre es am einfachsten, wenn der umgepumpte Volumenstrom den für die Umsetzung des DNT's erforderlichen Wasserstoff in gelöster Form enthielte. Leider kann das Reaktionsgemisch unter den vorliegenden Bedingungen (25 bar, 185°C) aber nur maximal 0,132 g/l Wasserstoff lösen, so daß ein Volumenstrom von 372 m³/h nur 49,1 kg Wasserstoff enthält. Da für die vollständige Umsetzung von 2000 kg/h DNT aber 132 kg/h Wasserstoff erforderlich sind, macht der gelöste Wasserstoff lediglich 37,2 % des erforderlichen aus, d.h., die fehlende Wasserstoffmenge von 82,9 kg/h wird am Reaktionsort nachgeliefert. Der Ejektor wird daher so betrieben, daß die hierfür notwendigen Stoffübergangsbedingungen gegeben sind. Unter den vorliegenden Reaktionsbedingungen (25 bar, 185 bis 195°C) hätte der Ejektor somit minimal 100 m³/h Wassserstoff anzusaugen, von denen sich 62,8 % im Mischrohr und der Test im nachfolgenden Behälter lösen müßte. Das Verhältnis des angesaugten Gasvolumenstroms zum umgepumpten Volumenstrom des Reaktionsgemisches beträgt also minimal 100:372 = 0,269. Da die zu dispergierende Gasmenge relativ klein ist, kann die gesamte Wasserstoffmenge von 132 kg/h ohne weiteres allein im Mischrohr des Ejektors gelöst werden. Um sicher zu sein, daß in dem umgepumpten Reaktionsgemisch keine Verarmung des gelösten Wasserstoffs auftritt, ist es zweckmäßig, eine größere als die unbedingt erforderliche Wasserstoffmenge von 100 m³/h anzusaugen. Selbst wenn man mit einem Umpumpstrom von 372 m³/h die 3fache H₂-Menge, also 300 m³/h ansaugt, können die Bedingungen im Mischrohr des Ejektors so gestaltet werden, daß sich die Wasserstoffmenge von 132 kg/h in diesem Teil der Reaktionsstrecke vollständig löst. Dies gelingt dadurch, daß der volumenbezogene Energieeintrag im Ejektor entsprechend eingestellt wird. Dieser Parameter, der die Intensität der Vermischung charakterisiert, kann in weiten Grenzen durch Wähl der Düsengeometrie variiert und den jeweiligen Erfordernissen angepaßt werden. Beispielsweise wird bei einem Umpumpstrom von 372 m³/h mit einer Düse von 70 mm Durchmesser eine Leistung von 37,3 kW eingetragen, die je nach Größe des Mischraums einen volumenbezogenen Energieeintrag von 2,9 bis 6,8 kW/l bewirkt. Auf die stündlich zu hydrierende DNT-Masse von 2 t bezogen, ergibt sich ein spezifischer Energieeintrag im Ejektor von 18,65 kW/t eingetragenem DNT.

## Patentansprüche

1. Kontinuierliches Verfahren zur Herstellung von aromatischen Di- und Polyaminen durch katalytische Hydrierung der entsprechenden aromatischen Di- und Polynitroverbindungen mit Wasserstoff unter Verwendung von Hydrierungskatalysatoren, dadurch gekennzeichnet, daß in einem Loop-Venturi-Reaktor mit Ejektor
a) in ein schnell fließendes Gemisch, das im wesentlichen aus aromatischem Di- oder Polyamin, feinteilig suspendiertem, festem Hydrierungskatalysator, Wasser und Wasserstoff besteht, ohne Lösungsmittel die entsprechende aromatische Di- oder Polynitroverbindung so eingetragen wird, daß
a1) die Di- oder Polynitroverbindung in dem Gemisch fein verteilt ist,
a2) das volumetrische Verhältnis des umgepumpten, schnell fließenden Gemisches zur eingetragenen Di- oder Polynitroverbindung 50 bis 500 beträgt,
a3) mit dem umgepumpten, schnell fließenden Gemisch der Ejektor so betrieben wird, daß im Mischraum des Ejektors eine Energie von 4 bis 40 kW/t eingetragener Di- oder Polynitroverbindung dissipiert wird,
a4) und ein Wasserstoffvolumenstrom im Verhältnis zum umgepumpten Gemischvolumen von 0,1 bis 7 angesaugt wird, wobei der benötigte Wasserstoff dem Gasraum des Reaktors entzogen wird und der bei der Reaktion verbrauchte Wasserstoff an beliebiger Stelle des Systems nachgeliefert wird,
b) das Reaktionsgemisch kontinuierlich aus dem System ausgetragen wird,
c) im Reaktor ein Druck von 5 bis 100 bar und eine Betriebstemperatur von 120-220°C aufrechterhalten werden und
d) gegebenenfalls die Reaktionswärme zur Dampferzeugung genutzt wird.

2. Verfahren gemäß Anspruch 1, dadurch gekennzeichnet daß ein Druck von 10 bis 50 bar aufrechterhalten wird.

3. Verfahren gemäß Anspruch 1 oder 2, dadurch gekennzeichnet, daß eine Betriebstemperatur von 150 bis 200°C aufrechterhalten wird.

4. Verfahren gemäß einem oder mehreren der Ansprüche 1 bis 3, dadurch gekennzeichnet, daß das Reaktionsgemisch unter Rückhalt des Katalysators aus dem System ausgetragen wird.

5. Verfahren gemäß einem oder mehreren der Ansprüche 1 bis 4, dadurch gekennzeichnet, daß die eingetragene, aromatische Di- oder Polynitroverbindung in reiner Form, als Mischung mit dem entsprechenden Di- oder Polyamin oder als Mischung mit dem entsprechenden Di- oder Polyamin und Wasser eingesetzt wird.

6. Verfahren gemäß einem oder mehreren der Ansprüche 1 bis 5, dadurch gekennzeichnet, daß als eingetragene aromatische Dinitroverbindung 2,4-Dinitrotoluol oder desssen technische Gemische mit 2,6-Dinitrotoluol eingesetzt werden.

## Claims

1. A continuous process for preparing aromatic di and polyamines by catalytic hydrogenation of the corresponding aromatic di and polynitro compounds with hydrogen using hydrogenation catalysts, characterised in that, in a loop-Venturi reactor with ejector,
a) the corresponding aromatic di or polynitro compound is introduced, without a solvent, into a rapidly flowing mixture which substantially consists of aromatic di or polyamines, finely divided suspended, solid hydrogenation catalyst, water and hydrogen, in such a way that
a1) the di or polynitro compound is finely distributed in the mixture,
a2) the ratio by volume of the recirculated, rapidly flowing mixture to the di or polynitro compound introduced is 50 to 500,
a3) the ejector operates on the recirculated rapidly flowing mixture in such a way that an energy of 4 to 40 kW/t of di or polynitro compound introduced is dissipated in the ejector mixing chamber,
a4) and the ratio of volume flow of hydrogen admitted to the recirculated mixing volume is 0.1 to 7, wherein the hydrogen required is drawn into the gas chamber of the reactor and the hydrogen consumed during reaction is supplied to any point in the system,
b) the reaction mixture is continuously withdrawn from the system,
c) a pressure of 5 to 100 bar and an operating temperature of 120-220°C are maintained in the reactor and
d) the heat of reaction is optionally used to produce steam.

2. A process according to Claim 1, characterised in that a pressure of 10 to 50 bar is maintained.

3. A process according to Claim 1 or 2, characterised in that an operating temperature of 150 to 200°C is maintained.

4. A process according to one or more of Claims 1 to 3, characterised in that the reaction mixture is withdrawn from the system with retention of the catalyst.

5. A process according to one or more of Claims 1 to 4, characterised in that the aromatic di or polynitro compound introduced is used in the pure form, as a mixture with the corresponding di or polyamine or as a mixture with the corresponding di or polyamine and water.

6. A process according to one or more of Claims 1 to 5, characterised in that 2,4-dinitrotoluene or its industrial grade mixture with 2,6-dinitrotoluene is used as the aromatic dinitro compound introduced.

## Revendications

1. Procédé en continu pour la préparation de di- et polyamines aromatiques par hydrogénation catalytique des composés di- et polynitro aromatiques correspondants avec de l'hydrogène en utilisant des catalyseurs d'hydrogénation, caractérisé en ce que, dans un réacteur Venturi en boucle équipé d'un éjecteur
a) on introduit, dans un mélange à écoulement rapide qui est constitué essentiellement par une di- ou polyamine aromatique, par un catalyseur d'hydrogénation solide mis en suspension à l'état finement divisé, par de l'eau et par de l'hydrogène, en l'absence de solvants, le composé di- ou polynitro aromatique correspondant de telle sorte que
a1) le composé di- ou polynitro est finement divisé dans le mélange,
a2) le rapport volumétrique du mélange à écoulement rapide recyclé par pompage au composé di- ou polynitro introduit s'élève de 50 à 500,
a3) on entraîne l'éjecteur avec le mélange à écoulement rapide recyclé par pompage de telle sorte que, dans la chambre de mélange de l'éjecteur, on obtient la dissipation d'une énergie de 4 à 40 kW/t du composé di- ou polynitro introduit,
a4) et on élimine par aspiration un courant volumique d'hydrogène de 0,1 à 7 rapporté au volume du mélange recyclé par pompage, l'hydrogène requis étant prélevé de l'espace du réacteur occupé par du gaz et l'oxygène consommé lors de la réaction étant restitué à n'importe quel endroit du système,
b) on évacue le mélange réactionnel en continu hors du système,
c) on maintient dans le réacteur une pression de 5 à 100 bar et une température de fonctionnement de 120-220°C, et
d) on tire éventuellement profit de la chaleur réactionnelle pour la formation de vapeur.

2. Procédé selon la revendication 1, caractérisé en ce qu'on maintient une pression de 10 à 50 bar.

3. Procédé selon la revendication 1 ou 2, caractérisé en ce qu'on maintient une température de fonctionnement de 150 à 200°C.

4. Procédé selon une ou plusieurs des revendications 1 à 3, caractérisé en ce qu'on évacue le mélange réactionnel hors du système en retenant le catalyseur.

5. Procédé selon une ou plusieurs des revendications 1 à 4, caractérisé en ce qu'on met en oeuvre le composé di- ou polynitro aromatique introduit, sous forme pure, sous forme d'un mélange avec la di- ou polyamine correspondante ou encore sous forme d'un mélange avec la di- ou la polyamine correspondante et de l'eau.

6. Procédé selon une ou plusieurs des revendications 1 à 5, caractérisé en ce qu'on met en oeuvre, à titre de composé dinitro aromatique introduit, le 2,4-dinitrotoluène ou ses mélanges techniques avec du 2,6-dinitrotoluène.
